# EUROPÄISCHE PATENTANMELDUNG

(11) **EP 1 795 159 A1**
(43) Veröffentlichungstag der Anmeldung: **13.06.2007**
(21) Anmeldenummer: 06024676.6
(22) Anmeldetag: 29.11.2006
(51) Int. Cl.: A61F 9/04

(54) **Augenschutzvorrichtung, insbesondere gegen Laserstrahlen und Lichtstrahlen hoher Intensität**

(30) Priorität: 09.12.2005 DE 102005058888
(71) Anmelder: LaserPoint AG, 59394 Nordkirchen (DE)
(72) Erfinder: Beyer, Antonius, 45731 Waltrop (DE)
(74) Vertreter: Gesthuysen, von Rohr & Eggert

(57) **Zusammenfassung**

Die Erfindung betrifft eine Augenschutzvorrichtung (1), bestehend aus zwei innen konkaven und außen konvexen Schutzschalen (2, 3), welche die Augen und die Augenlider einer Trägerperson abdecken und durch einen elastischen Drahtbügel (8) verbunden sind, dessen Enden (6, 7) beabstandet vom Außenrand (2c, 3c) an den Schutzschalen (2, 3) an einem Vorsprung (4, 5) befestigt sind.

Der Erfindung liegt die Aufgabe zugrunde, eine Augenschutzvorrichtung der eingangs genannten Gattung zu schaffen, die den als unangenehm empfundenen, scharfrandigen, mechanischen Druck des Außenrandes der Schutzschalen nicht mehr aufweist und trotzdem eine sichere Abdichtung des Außenrandes der Schutzschalen gegenüber der Gesichtshaut gewährleistet.

Diese Aufgabe wird erfindungsgemäß dadurch gelöst, dass jede Schutzschale (2, 3) an ihrem Außenrand (2c, 3c) von einem lichtundurchlässigen, entropieelastischen Ring (9,10) mit hoher Wärmebeständigkeit vollständig umgriffen ist und die bandförmige Halterungseinrichtung (14) mit einer Verstell- und einer Arretierungsvorrichtung (15) versehen ist, mit welcher die ungespannte Länge der Halterungseinrichtung (14) rasch zu verlängern oder zu verkürzen ist, wobei die Halterungseinrichtung (14) in Verbindung mit den entropieelastischen Ringen (9, 10) der Schutzschalen (2, 3) eine gemeinsame Feder (9, 10, 14) bildet, deren Federvorspannung über die Verstell- und Arretierungsvorrichtung (15) der mechanischen Druckverträglichkeit der Trägerperson angepaßt ist.

## Beschreibung

Die Erfindung betrifft eine Augenschutzvorrichtung, insbesondere gegen Laser- und Lichtstrahlen, bestehend aus zwei innen konkaven und außen konvexen Schutzschalen, welche die Augen und die Augenlider einer Trägerperson abdecken und durch einen elastischen Drahtbügel verbunden sind, dessen Enden beabstandet vom Außenrand an den Schutzschalen an einem Vorsprung befestigt sind und dessen Mittelteil einen deutlichen Abstand zur Nase einhält, wobei die Schutzschalen mittels einer elastischen Halterungseinrichtung am Kopf der Trägerperson gehalten sind.

Eine bekannte Augenschutzvorrichtung dieser Art ist aus der DE 101 06 668 C2 bekannt geworden. Bei dieser Ausführungsform besteht der Drahtbügel aus einem dünnen, bruchfesten sowie hochverwindungselastischen Draht aus einer Titan-Nickel-Legierung, dessen freie Enden mit der konvexen Außenseite der vollständig geschlossenen Schutzschalen durch Laserschweißung, Löten oder Kleben unlösbar verbunden sind.

Derartige Augenschutzvorrichtungen finden nicht nur Anwendung bei nicht invasiven Eingriffen auf der Haut, sondern auch bei Hautbehandlungen durch mehrfarbiges Licht von hoher Intensität.

Eine weitere Augenschutzvorrichtung der eingangs genannten Art ist in der US-PS 2,283,752 offenbart. Dabei sind die Enden des Drahtbügels zu einer Öse verformt, die an einer die jeweilige Schutzschale im Mittenbereich durchdringenden Nietverbindung gehalten ist. Zum Zeitpunkt der Veröffentlichung dieser Druckschrift gab es noch keine Laserstrahlen und daher auch noch keine Laserstrahlen-Operationen, jedoch hätte auch diese Augenschutzvorrichtung dafür eingesetzt werden können, weil ihre Schutzschalen aus Metall hergestellt werden konnten. Diese vorbekannte Augenschutzvorrichtung ist mit dem Nachteil behaftet, daß jede Schutzschale von einer Nietverbindung durchdrungen ist und deshalb ein seitlicher Laserstrahleinfall befürchtet werden muß. Das gilt insbesondere dann, wenn sich mit voranschreitendem Gebrauch die Ösenverbindungen der Enden des Drahtbügels lockern oder aufweiten.

Eine weitere, jedoch gattungsfremde Augenschutzvorrichtung ist aus der US-PS 5,918,600 der inhaltsgleichen WO 89/53775 bekannt geworden. Dabei ist der U-förmige, elastische Drahtbügel an seinen Enden über eine zylindrische Spirale mit dem Außenrand der Schutzschalen verbunden, wobei die Spiralen auf starren Haken aufgesetzt und mit diesen an einem Außenrand der Schutzschale fest verbunden sind. An dem gegenüberliegenden Außenrand der Schutzschalen sind entweder Haken oder gleichfalls von einer zylindrischen Spirale gewundene, endseitig mit einem Haken versehene Befestigungsvorsprünge angeordnet, an denen ein elastisches Band als Halterungseinrichtung eingehakt wird. Diese Augenschutzvorrichtung ist zwar mit geschlossenen Schutzschalen versehen, jedoch mit dem Nachteil verbunden, daß der starr über die Spiralen an den Haken befestigte Drahtbügel relativ leicht abbricht und bei Personen mit einem großen Augenabstand der vom Drahtbügel abgewandte Außenbereich der Schutzschalen beim Aufbiegen des Drahtbügels nach außen abgeklappt und durch das Zugband entsprechend wieder gegen den die Augen und die Augenlider umgebenden Gesichtsbereich der betreffenden Trägerperson unter entsprechenden Zugkräften herangezogen werden muß. Dadurch entstehen durch die Befestigungshaken an dem Außenbereich ebenso durch die Federhaken am Innenbereich punktuelle Belastungen, die von der betreffenden Trägerperson während des chirurgischen Eingriffs als äußerst unangenehm empfunden werden.

Sämtliche drei Ausführungsformen haben den gemeinsamen Nachteil, daß die harten, aus Metall bestehenden Schutzschalen scharfrandig ausgebildet und mit entsprechendem mechanischem Druck im Bereich der Augenumgebung sich in die Gesichtshaut "eingraben". Dies wird von der Trägerperson als äußerst unangenehm empfunden und kann dazu führen, daß diese während der Operation die Schutzschalen verschiebt und dadurch eine Lichtleckage zwischen den Schutzschalen und der Gesichtshaut zum Eintritt von schädlichen Laserstrahlen entsteht. Ein solcher Lichtspalt kann auch dadurch entstehen, daß beim Aufsetzen des Außenrandes der harten Schutzschalen auf Gesichtsfalten, Narben, Warzen oder sonstigen Hautunebenheiten ein Spalt offen bleibt und zu einer Lichtleckage führt.

Hier setzt die Erfindung ein. Dieser liegt die Aufgabe zugrunde, eine Augenschutzvorrichtung der eingangs genannten Gattung zu schaffen, die den als unangenehm empfundenen, scharfrandigen, mechanischen Druck des Außenrandes der Schutzschalen nicht mehr aufweist und trotzdem eine sichere Abdichtung des Außenrandes der Schutzschalen gegenüber Leckagen sowie einen angenehm empfundenen Sitz der Schutzschalen auf den abzudichtenden Gesichtshautpartien gewährleistet.

Diese Aufgabe wird in Verbindung mit dem eingangs genannten Gattungsbegriff erfindungsgemäß dadurch gelöst, daß jede Schutzschale an ihrem Außenrand von einem lichtundurchlässigen, entropieelastischen Ring mit hoher Wärmebeständigkeit vollständig umgriffen und die bandförmige Halterungseinrichtung mit einer Verstell- und einer Arretierungsvorrichtung versehen ist, mit welcher die ungespannte Länge der Halterungseinrichtung rasch zu verlängern oder zu verkürzen ist, wobei die Halterungseinrichtung in Verbindung mit den entropieelastischen Ringen der Schutzschalen eine gemeinsame Feder bildet, deren Federvorspannung über die Verstell- und Arretierungsvorrichtung der mechanischen Druckverträglich der Trägerperson angepaßt ist.

Durch diese Ausbildung werden erstmalig Schutzschalen geschaffen, die ihre Auflageposition von der Trägerperson des entropieelastischen Ringes angenehm empfunden werden und aufgrund ihrer gegenüber dem schmalen Außenrand der Schutzschalen größeren Anlagefläche die Anpresskraft pro Flächeneinheit und damit den Anpreßdruck vermindern und gemeinsam mit der bandförmigen Halterungseinrichtung sowie der Verstell- und Arretierungsvorrichtung eine Federvorspannung zulassen, die trotzdem eine absolute Lichtdichtheit ohne jede Leckagen der Schutzschalen auf der Gesichtshaut der Trägerperson gewährleistet und rasch der Druckverträglichkeit der Trägerperson angepaßt werden kann.

Nach einer besonders vorteilhaften Weiterbildung der Erfindung weisen die entropieelastischen Ringe an ihrem dem Außenrand der Schutzschalen zugekehrten Innenrand eine umlaufende Nut auf, mit welcher sie in ihrer Endposition unter einer entropieelastischen Federvorspannung den Außenrand der Schutzschalen übergreifen. Dadurch wird ein sicherer Sitz der entropieelastischen Ringe auf den aus Metall bestehenden Schalen und eine federelastische Anpassung der Schutzschalen an Hautunebenheiten gewährleistet.

Nach einer besonders vorteilhaften Weiterbildung der Erfindung bestehen die entropieelastischen Ringe für die Schutzschalen aus einem Silikon, das über vier Stunden eine Temperaturfestigkeit von 200 °C aufweist.

Diese entropieelastischen Ringe sind sowohl mit chemischen Desinfektionsmitteln als auch mit thermischer Desinfektion in einem Autoklaven sterilisierbar und bei Verschleiß werkzeuglos austauschbar. Dadurch wird auch die im OP-Bereich unabdingbar notwendige Sterilität sichergestellt.

Die Verstell- und Arretierungsvorrichtung besteht vorteilhaft aus einem kugelförmigen oder zylindrischen Körper mit einer zentralen Durchgangsöffnung, durch welche die bandförmige Halterungseinrichtung doppelbahnig durch eine Verengung als Verstell- und Arretierungsvorrichtung durchgezogen ist.

Alternativ wird die Verstell- und Arretierungsvorrichtung von einem Schikanevorsprung innerhalb der Durchgangsöffnung gebildet.

An der bandförmigen Halterungseinrichtung ist ein weiterer kugel- oder zylinderförmiger Körper aufgezogen, um ein Durchschleifen der bandförmigen Halterungseinrichtung durch die Verstell- und Arretierungsvorrichtung zu verhindern.

Das Band der Halterungseinrichtung besteht vorteilhaft aus einer nicht brennbaren, sondern nur verkokbaren Seide und Gummibändern, deren Enden zu einer Schlaufe gewunden und von einem Schrumpfschlauch aus einem Polyolefin mit einem Innenkleber unverstellbar zusammengehalten sind. Dadurch wird eine Verminderung der Federvorspannung von entropieelastischen Ringen und Halterungseinrichtung durch eine unerwünschte Erweiterung der Schlaufenlänge sicher unterbunden.

Die unverstellbaren Schlaufen werden in an sich bekannter Weise jeweils von einem Haken gehalten, der beabstandet vom Außenrand der Schutzschalen fest mit diesen verbunden ist.

Nach einer vorteilhaften Weiterbildung der Erfindung sind die Enden des Drahtbügels von einer zylindrischen Hülse als Vorsprung der Schutzschalen in einem Schwenklager gehaltert. Dieses Schwenklager besteht aus einer Kunststoffhülse aus PTFE (Polytetrafluorethylen), die in eine zylindrische Bohrung der zylindrischen Hülse ortsfest eingefügt ist. Da PTFE ein hochwarmfester Kunststoff mit einer Temperaturbeständigkeit bis 250 °C ist, können die Augenschutzschalen mit dem Drahtbügel nach Abnahme der Verstell- und Arretierungsvorrichtung komplett in einem Autoklaven thermisch sterilisiert werden.

Der Drahtbügel besteht in an sich bekannter Weise aus einem dünnen, bruchfesten sowie bruchverbindungselastischen Draht aus einer Titan-Nickel-Legierung. In seinem Mittelteil ist er entweder U-förmig, trapezförmig, dreieckförmig oder mehreckförmig ausgebildet.

Ein Ausführungsbeispiel der Erfindung ist in den Zeichnungen dargestellt. Dabei zeigen:
Fig. 1 die Draufsicht auf die Augenschutzvorrichtung,
Fig. 2 eine vergrößerte Ansicht des Vorsprunges auf der rechten Augenschale mit dem darin schwenkbeweglich gehalterten Ende des Drahtbügels,
Fig. 3 eine vergrößerte Ansicht des Vorsprunges auf der linken Augenschale mit dem darin gehalterten anderen Ende des Drahtbügels,

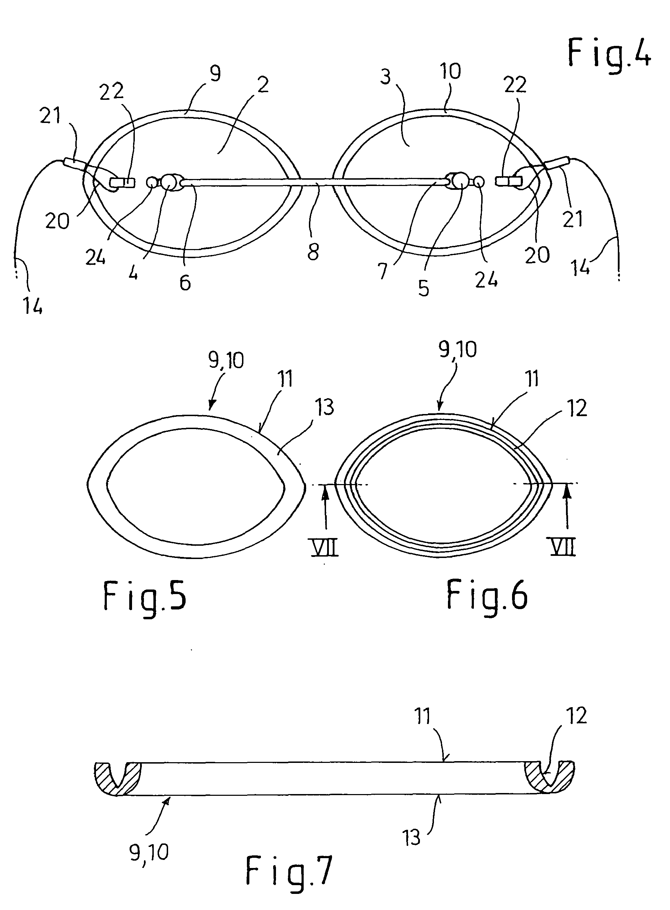

Fig. 4 die Ansicht auf die Augenschutzvorrichtung in Richtung des Pfeiles IV von Fig. 1 ,
Fig. 5 die Vorderansicht eines entropieelastischen Ringes,
Fig. 6 die Rückansicht eines entropieelastischen Ringes mit der Nut,
Fig. 7 die Schnittansicht entlang der Linie VII/VII von Fig. 6 in vergrößerter Darstellung,
Fig. 8 die Augenschutzvorrichtung in Position auf dem Augenbereich einer Trägerperson mit zur Nase hin verschwenktem Drahtbügel,
Fig. 9 eine perspektivische Draufsicht in Richtung des Pfeiles IX von Fig. 1 auf die Augenschutzvorrichtung,
Fig. 10 die Augenschutzvorrichtung in Position auf dem Gesichtsbereich einer Trägerperson mit vom Nasenbereich in Richtung Stirn hochgeklapptem Drahtbügel,
Fig. 11 einen Diametralschnitt durch die Verstell- und Arretierungsvorrichtung in Form einer Kugel mit einem Verengungsbereich in vergrößerter Darstellung des Bereiches von Fig. 1 und
Fig. 12 eine weitere Ausführungsform der kugelförmigen Verstell- und Arretierungsvorrichtung im Diametralschnitt mit einem Schikanevorsprung zur Verengung des Durchtrittsquerschnittes für die Halterungseinrichtung in vergrößerter Darstellung des Bereichs XII von Fig. 1.

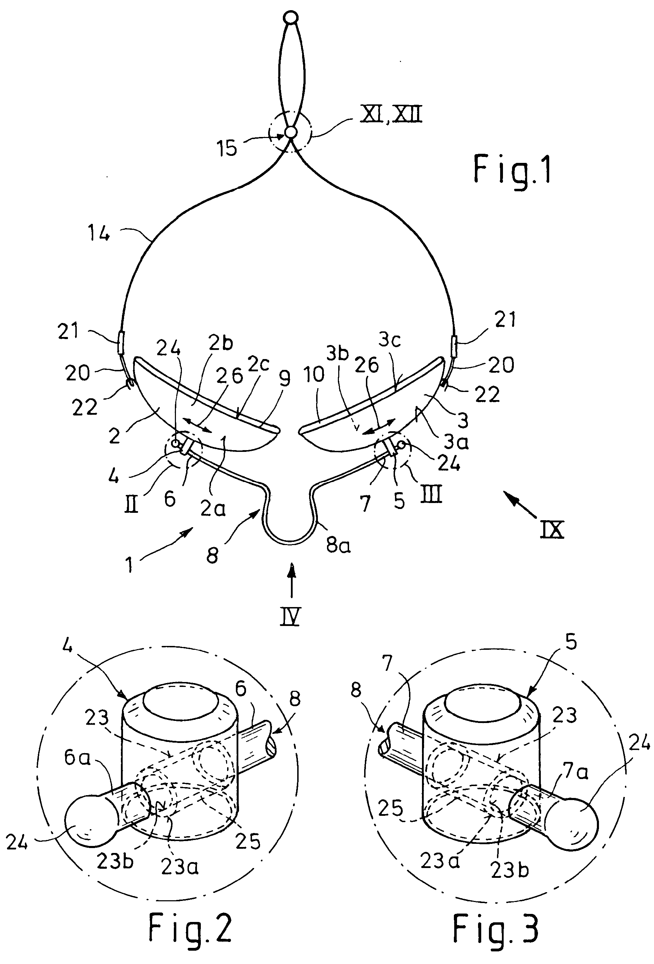

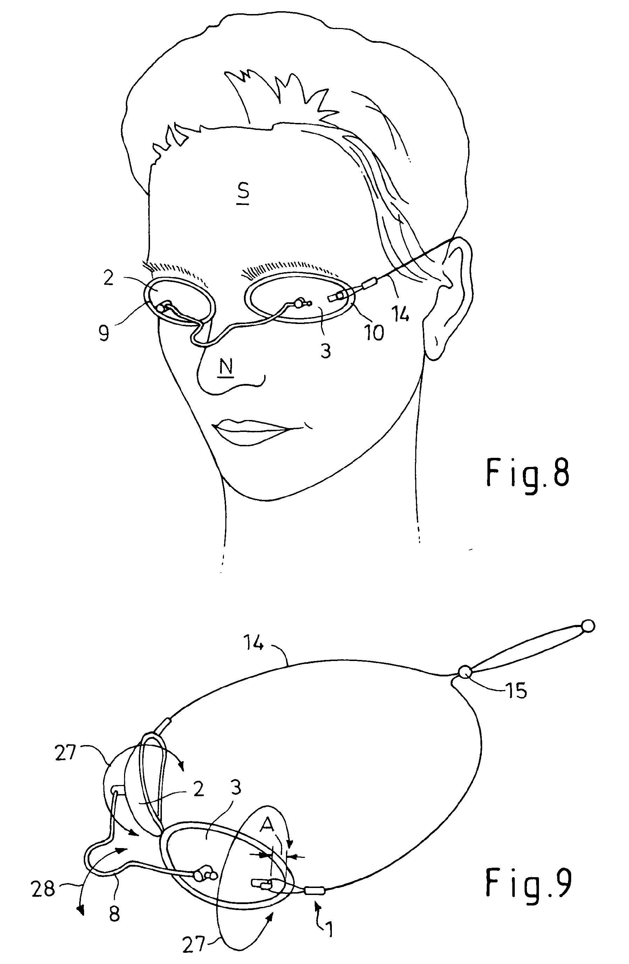

Die Augenschutzvorrichtung 1 gemäß den Figuren 1 und 9 besteht im wesentlichen aus zwei Schutzschalen 2 und 3 aus Metall oder Keramik, die in ihrem Mittenbereich je einen Vorsprung 4, 5 aufweisen, an welchem die Enden 6, 7 eines entropieelastischen Drahtbügels 8 schwenkbeweglich gehaltert sind. Die Schutzschalen 2, 3 sind auf ihrer Außenseite 2a, 3a konvex und auf ihrer Innenseite 2b, 3b konkav gestaltet. Die Vorsprünge 4, 5 weisen einen deutlichen Abstand von den Außenrändern 2c und 3c der Schutzschalen 2 und 3 auf.

Weiterhin sind die Schutzschalen 2 und 3 an ihren Außenrändern 2c und 3c von je einem entropieelastischen Ring 9, 10 umgriffen, der mit seinem dem Außenrand 2c, 3c zugekehrten Innenrand 11 mit einer umlaufenden Nut 12 versehen ist, mit welcher er in seiner Endposition unter einer entropieelastischen Federspannung den Außenrand 2c, 3c der Schutzschalen 2, 3 übergreift. Der Außenrand 13 der entropieelastischen Ringe 9, 10 ist konvex ausgebildet.

Die entropieelastischen Ringe 9, 10 für die Schutzschalen 2, 3 bestehen aus einem Silikon, welches vorteilhaft über vier Stunden eine Temperaturfestigkeit von 200 °C aufweist. Dadurch können die entropieelastischen Ringe 9, 10 nicht nur in einer Autoklaven sterilisiert werden, sondern sich auch dann als beständig erweisen, wenn sie während eines chirurgischen Eingriffes von einem Laserstrahl getroffen werden sollten.

Außerdem ist die Augenschutzvorrichtung 1 mit einer bandförmigen Halterungseinrichtung 14 sowie mit einer Verstell- und Arretierungsvorrichtung 15 versehen.

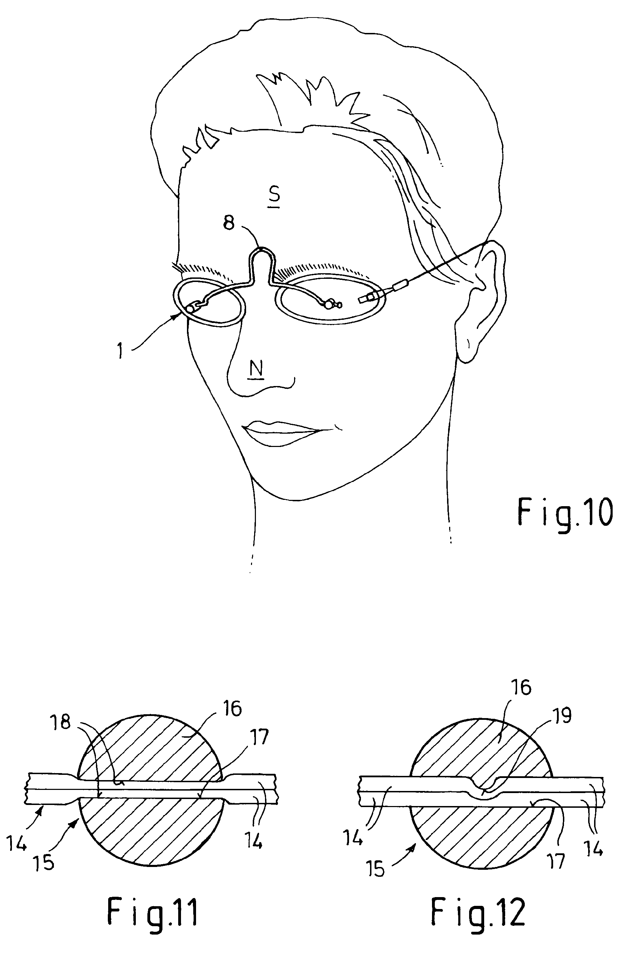

Die Verstell- und Arretierungsvorrichtung 15 besteht gemäß den Fig. 11 und 12 aus einem kugelförmigen oder zylindrischen Körper 16 mit einer zentralen Durchgangsöffnung 17, durch welche die bandförmige Halterungseinrichtung 14 doppelbahnig durch eine Verengung durchgezogen ist, die gemäß Fig. 12 einen Schikanevorsprung 19 innerhalb der Durchgangsöffnung 17 aufweisen kann. Diese Verstell- und Arretierungsvorrichtung 15 besteht vorteilhaft aus POM (Polyacetal) und weist einen thermischen Einsatzbereich in Luft bis 80 °C auf.

Das Band der Halterungseinrichtung 14 wird vorteilhaft aus einer Kombination von nicht brennbarer, sondern nur verkokbarer Seide oder Kunstseide in Verbindung mit Gummibändern hergestellt, deren Enden zu einer Schlaufe 20 gewunden und mit einem Schrumpfschlauch 21 aus einem Polyolefin mit einem Innenkleber unverstellbar zusammengehalten sind. Die Temperaturbeständigkeit des Schrumpfschlauches 21 reicht von -55 °C bis + 110 °C und seine Schrumpftemperatur beträgt ca. + 120 °C.

Die Schlaufen 20 der Halterungseinrichtung 14 sind an Haken 22 aufgezogen, die auf der Außenseite 2a, 3a der Schutzschalen 2, 3 mit einem Abstand A (siehe Fig. 9) vom Außenrand 2c, 3c der Schutzschalen 2, 3 durch Aufschweißen, Löten oder Kleben befestigt sind und aus dem gleichen Werkstoff wie die Schutzschalen 2, 3 bestehen. Dadurch kann die Halterungseinrichtung 14 abgenommen und getrennt davon die Schutzschalen 2, 3 mit den Drahtbügeln 8 sowie einschließlich der entropieelastischen Ringe 9, 10 in einem Autoklaven sterilisiert werden, wohingegen die Verstell- und Arretierungsvorrichtung 15 mit der Halterungseinrichtung 14 einer chemischen Desinfektion unterzogen wird.

Je nach Einsatzort des chirurgischen Eingriffes im Gesichtsbereich mittels eines Laserstrahles kann der Drahtbügel 8 hinderlich sein. Soll beispielsweise dieser chirurgische Eingriff im Nasenbereich stattfinden, wird der Drahtbügel 8 gemäß Fig. 10 zur Stirn S der Trägerperson hochgeklappt, ohne jedoch die Stirn S der Trägerperson zu kontaktieren. Wenn hingegen der chirurgische Eingriff mittels eines Laserstrahles im Nasenwurzel- bzw. im Stirnbereich S der Trägerperson stattfindet, wird der Drahtbügel 8 gemäß Fig. 8 zur Nase N heruntergeklappt, ohne jedoch die Nase N zu berühren. Bei dieser Schwenkbewegung des Drahtbügels 8 werden die von der Halterungseinrichtung 14 gehaltenen Schutzschalen 2, 3 nicht in ihrer Position beeinträchtigt, weil die Enden 6, 7 des Drahtbügels 8 im Schwenklager 25 der Vorsprünge 4, 5 gelagert sind. Diese Schwenklagerung ist aus den Fig. 2 und 3 ersichtlich.

Gemäß Fig. 2 durchgreift das Ende 6 des Drahtbügels 8 eine abgestufte, zylindrische Bohrung 23, deren Teil der Bohrung 23a mit größerem Durchmesser dem Drahtbügel 8 und deren Teil 23b mit kleinerem Durchmesser dem freien Ende 6a zugekehrt ist, welches mit einer Verdickung 24 versehen ist, um ein Herausziehen des Endes 6 aus dem Vorsprung 4 zu unterbinden. In dem Teil 23a mit dem größeren Durchmesser ist als Schwenklager 4, 5, 25 eine zylindrische Kunststoffhülse 25 aus PTFE (Polytetrafluorethylen) ortsfest eingefügt. Die Vorsprünge 4, 5 in Form der zylindrischen Hülsen bestehen aus einer Chrom-Nickel-Legierung, die etwa dem Material der Schutzschalen 2, 3 entspricht.

Der Drahtbügel 8 wird in an sich bekannter Weise aus einem dünnen, bruchfesten sowie hochverwindungselastischen Draht aus einer Titan-Nickel-Legierung hergestellt, um auf diese Weise selbst bei extremen Verwindungsbelastungen ein Auseinanderfallen der Schutzschalen 2, 3 auszuschließen.

Um einen korrekten Augenabstand der Schutzschalen 2, 3 einzustellen, können die Enden 6, 7 des Drahtbügels 8 in beiden Richtungen des Doppelpfeiles 26 gemäß Fig. 1 verschoben und sowohl der Drahtbügel 8 als auch die Schutzschalen 2, 3 gemäß Fig. 9 in Richtung der Doppelpfeile 27, 28 relativ zueinander verschwenkt werden.

Der Kern der Erfindung beruht darin, daß nunmehr die entropieelastischen Ringe 9, 10 der Schutzschalen 2, 3 in Verbindung mit der elastischen Halterungseinrichtung 14 eine gemeinsame Feder bilden, deren Federvorspannung über die Verstell- und Arretierungsvorrichtung 15 der mechanischen Druckverträglichkeit der Trägerperson in individueller Weise angepaßt werden kann. Denn sowohl metallische als auch keramische Werkstoffe der Schutzschalen 2, 3 sind mit dem Nachteil verbunden, daß sie beim Aufsetzen auf den Augenbereich als kalt empfunden werden und somit vom Gesichtsbereich zunächst aufgewärmt werden müssen. Aufgrund dieses unangenehmen Druck- und Temperaturempfmdens ist jede Trägerperson bemüht, diese durch mechanische Verschiebung zu kompensieren. Dadurch aber wiederum können die Schutzschalen 2, 3 in eine Position verschoben werden, in welche zwischen den Außenrändern 2c, 3c der Schutzschalen 2, 3 und der damit kontaktierenden Gesichtshaut ein Leckagespalt entstehen kann, der einen Laserstrahl durchläßt. Dies wird nunmehr von den entropieelastischen Ringen 9, 10 in Verbindung mit der ebenso elastischen Halterungseinrichtung 14 unterbunden, weil sich die vorteilhaft aus Silikon bestehenden Ringe 9, 10 der jeweiligen Gesichtskontur der Trägerperson in angenehmer Weise anpassen und somit Unebenheiten, wie Falten, Narben, Warzen oder dergleichen im Kontaktbereich kompensieren.

Da außerdem Silikon eine ausgezeichnete Hautverträglichkeit aufweist, wird der Kontakt der entropieelastischen Ringe 9, 10 auf der Haut als angenehm empfunden, so daß die Trägerperson während des chirurgischen Eingriffes in einer stabilen Operationslage verharren kann.

Und schließlich trägt die optimale Verstellbarkeit der Schutzschalen 2, 3 und des Drahtbügels 8 gemäß den Pfeilen 27, 28 von Fig. 9 sowie die Verstellbarkeit der Augenschalen 2, 3 auf den Enden 6, 7 des Drahtbügels 8 in Richtung des Doppelpfeiles 26 gemäß Fig. 1 zur Erzielung eines korrekten Augenabstandes zu einem optimalen Sitz und damit zu einer gesteigerten Sicherheit gegen Lichtleckagen bei.

### Bezugszeichenliste:

| | |
|---|---|
| Augenschutzvorrichtung | 1 |
| Schutzschalen | 2,3 |
| konvexe Außenseite der Schutzschalen 2, 3 | 2a, 3a |
| konkave Innenseite der Schutzschalen 2, 3 | 2b, 3b |
| Außenrand der Schutzschalen 2, 3 | 2c, 3c |
| Vorsprünge | 4, 5 |
| Enden von Drahtbügel 8 | 6, 7 |
| Drahtbügel | 8 |
| entropieelastische Ringe | 9, 10 |
| Innenseite d. entropieelastischen Ringe | 11 |
| umlaufende Nut der entropieelastischen Ringe | 12 |
| Außenseite der entropieelastischen Ringe | 13 |
| Halterungseinrichtung | 14 |
| Verstell- und Arretierungsvorrichtung | 15 |
| Kugel- oder zylindrischer Körper der Verstell- und Arretierungsvorrichtung 15 | 16 |
| Durchgangsöffnung in der Verstell- und Arretierungsvorrichtung 15 | 17 |
| Verengung der Verstell- und Arretierungsvorrichtung 15 | 18 |
| Schikanevorsprung in der Durchgangsöffnung 17 | 19 |
| Schlaufen der Halterungseinrichtung 14 | 20 |
| Schrumpfschlauch auf den Schlaufenenden | 21 |
| Haken für die Halterungseinrichtung 14 | 22 |
| zylindrische Bohrung in Hülsen 4, 5 | 23 |
| Bohrung mit größerem Durchmesser | 23a |
| Bohrung mit kleinerem Durchmesser | 23b |
| Verdickung an den Enden des Drahtbügels 8 | 24 |
| zylindrische Kunststoffhülse | 25 |
| Doppelpfeile | 26, 27, 28 |
| Abstand der Haken 22 vom Außenrand 2c, 3c | A |
| Stirn der Trägerperson | S |
| Nase der Trägerperson | N |

## Patentansprüche

1. Augenschutzvorrichtung, insbesondere gegen Laser- und Lichtstrahlen, bestehend aus zwei innen konkaven und außen konvexen Schutzschalen, welche die Augen und die Augenlider einer Trägerperson abdecken und durch einen elastischen Drahtbügel verbunden sind, dessen Enden beabstandet vom Außenrand an den Schutzschalen an einem Vorsprung befestigt sind und dessen Mittelteil einen deutlichen Abstand zur Nase einhält, wobei die Schutzschalen mittels einer elastischen Halterungseinrichtung am Kopf der Trägerperson gehalten sind, **dadurch gekennzeichnet, dass** jede Schutzschale (2, 3) an ihrem Außenrand (2c, 3c) von einem lichtundurchlässigen, entropieelastischen Ring (9,10) mit hoher Wärmebeständigkeit vollständig umgriffen ist und die bandförmige Halterungseinrichtung (14) mit einer Verstell- und einer Arretierungsvorrichtung (15) versehen ist, mit welcher die ungespannte Länge der Halterungseinrichtung (14) rasch zu verlängern oder zu verkürzen ist, wobei die Halterungseinrichtung (14) in Verbindung mit den entropieelastischen Ringen (9, 10) der Schutzschalen (2, 3) eine gemeinsame Feder (9, 10, 14) bildet, deren Federvorspannung über die Verstell- und Arretierungsvorrichtung (15) der mechanischen Druckverträglichkeit der Trägerperson angepaßt ist.

2. Augenschutzvorrichtung nach Anspruch 1, **dadurch gekennzeichnet, dass** die entropieelastischen Ringe (9, 10) an ihrem dem Außenrand (2c, 3c) der Schutzschalen (2, 3) zugekehrten Innenrand (11) eine umlaufende Nut (12) aufweisen, mit welcher sie in ihrer Endposition unter einer entropieelastischen Federvorspannung den Außenrand (2c, 3c) der Schutzschalen (2, 3) übergreifen.

3. Augenschutzvorrichtung nach Anspruch 1 oder 2, **dadurch gekennzeichnet, dass** die entropieelastischen Ringe (9, 10) für die Schutzschalen (2, 3) aus einem Silikon bestehen.

4. Augenschutzvorrichtung nach Anspruch 3, **dadurch gekennzeichnet, dass** das Silikon über vier Stunden eine Temperaturfestigkeit von 200 °C aufweist.

5. Augenschutzvorrichtung nach Anspruch 3 oder 4, **dadurch gekennzeichnet, dass** die entropieelastischen Ringe (9, 10) in einem Autoklaven sterilisierbar sind.

6. Augenschutzvorrichtung nach einem der Ansprüche 1 bis 5, **dadurch gekennzeichnet, dass**dieVerstell-und Arretierungsvorrichtung (15) aus einem Kugel- oder zylindrischen Körper (16) mit einer zentralen Durchgangsöffnung (17) besteht, durch welche die bandförmige Halterungseinrichtung (14) doppelbahnig durch eine Verengung (18) als Verstell- und Arretierungseinrichtung (15) durchgezogen ist.

7. Augenschutzvorrichtung nach einem der Ansprüche 1 bis 6, **dadurch gekennzeichnet, dass** die Verstell- und Arretierungseinrichtung (15) mit einem Schikanevorsprung (19) innerhalb der Durchgangsöffnung (17) versehen ist.

8. Augenschutzvorrichtung nach einem der Ansprüche 1 bis 7, **dadurch gekennzeichnet, dass** die Verstell- und Arretierungseinrichtung (15) aus POM (Polyacetal) besteht und einen thermischen Einsatzbereich in Luft bis 80 °C aufweist.

9. Augenschutzvorrichtung nach einem der Ansprüche 1 bis 8, **dadurch gekennzeichnet, dass** das Band der Halterungseinrichtung (14) aus einer Kombination von nicht brennbaren, sondern nur verkokbaren Seide und Gummibändern besteht, deren Enden zu einer Schlaufe (20) gewunden und mit von einem Schrumpfschlauch (21) aus einem Polyolefin mit einem Innenkleber unverstellbar zusammengehalten sind.

10. Augenschutzvorrichtung nach Anspruch 9, **dadurch gekennzeichnet, dass** die Temperaturbeständigkeit des Schrumpfschlauches (21) von -55 °C bis + 110 °C reicht und seine Schrumpftemperatur +120 °C beträgt.

11. Augenschutzvorrichtung nach Anspruch 9 und 10, **dadurch gekennzeichnet, dass**dieunverstellbaren Schlaufen (20) in an sich bekannter Weise von einem Haken (22) gehalten sind, der beabstandet vom Außenrand (2c, 3c) der Schutzschalen (2, 3) fest mit diesen verbunden ist.

12. Augenschutzvorrichtung nach einem der Ansprüche 1 bis 11, **dadurch gekennzeichnet, dass**die Enden (6, 7) des Drahtbügels (8) in einer zylindrischen Hülse (4, 5) als Vorsprung der Schutzschalen (2, 3) in einem Schwenklager (4, 5, 25) gehaltert sind.

13. Augenschutzvorrichtung nach Anspruch 12, **dadurch gekennzeichnet, dass** das Schwenklager (4, 5, 25) aus einer Kunststoffhülse (25) aus PTFE (Polytetrafluorethylen) besteht, die in eine zylindrische Bohrung (23a) der zylindrischen Hülse (4, 5) ortsfest eingefügt ist.

14. Augenschutzvorrichtung nach Anspruch 13, **dadurch gekennzeichnet, dass** die zylindrische Bohrung (23) abgestuft ist und in dem Teil der Bohrung (23a) mit dem größeren Durchmesser die Kunststoffhülse (25) eingesetzt ist und in dem Teil (23b) mit dem kleineren Durchmesser das Ende (6, 7) des Drahtbügels (8) geführt und an seinem freien Ende (6a, 7a) mit einer den kleineren Durchmesser der Bohrung (23b) übersteigenden Verdickung (24) versehen ist.

15. Augenschutzvorrichtung nach einem der Ansprüche 12 bis 14, **dadurch gekennzeichnet, dass** die zylindrischen Hülsen (4, 5) aus einer Chrom-Nickel-Legierung bestehen und auf etwa die Mitte der Schutzschalen (2, 3) aufgelötet, aufgeschweißt oder aufgeklebt sind.

16. Augenschutzvorrichtung nach einem der Ansprüche 1 bis 15, **dadurch gekennzeichnet, dass**der Drahtbügel (8) in an sich bekannter Weise aus einem dünnen, bruchfesten sowie hochverwindungselastischen Draht aus einer Titan- Nickel-Legierung besteht.

17. Augenschutzvorrichtung nach einem der Ansprüche 1 bis 16, **dadurch gekennzeichnet, dass** der Drahtbügel (8) in seinem Mittelteil U-förmig, trapezförmig, dreieckförmig oder mehreckförmig ausgebildet ist.
